# EUROPEAN PATENT SPECIFICATION

(11) **EP 3 655 762 B1**
(45) Date of publication and mention of the grant of the patent: **07.04.2021**
(21) Application number: 18749577.5
(22) Date of filing: 18.07.2018
(51) Int. Cl.: G01N 21/51, G01N 33/569

(54) **METHOD OF DETECTING MYCOBACTERIA**
VERFAHREN ZUR DETEKTION VON MYKOBAKTERIEN
PROCÉDÉ DE DÉTECTION DE MYKOBACTÉRIES

(30) Priority: 18.07.2017 IT 201700081630
(43) Date of publication of application: 27.05.2020
(73) Proprietor: Alifax S.r.l., 35020 Polverara (PD) (IT)
(72) Inventor: GALIANO, Paolo, 35100 Padova (IT)
(74) Representative: Petraz, Gilberto Luigi
(86) International application number: PCT/IT2018/050133
(87) International publication number: WO 2019/016842

(56) References cited:
- N. HASEGAWA ET AL: "New Simple and Rapid Test for Culture Confirmation of Mycobacterium tuberculosis Complex: a Multicenter Study", JOURNAL OF CLINICAL MICROBIOLOGY, vol. 40, no. 3, 1 March 2002 (2002-03-01), pages 908-912, XP055453345, US ISSN: 0095-1137, DOI: 10.1128/JCM.40.3.908-912.2002
- LG WAYNE: "DYNAMICS OF SUBMERGED GROWTH OF MYCOBACTERIUM TUBERCULOSIS UNDER AEROBIC AND MICROAEROPHILIC CONDITIONS", THE AMERICAN REVIEW OF RESPIRATORY DISEASE, AMERICAN THORACIC SOCIETY, US, vol. 114, no. 4, 1 January 1976 (1976-01-01), pages 807-811, XP000915495, ISSN: 0003-0805
- KATIA PENUELAS-URQUIDES ET AL: "Measuring of Mycobacterium tuberculosis growth: a correlation of the optical measurements with colony forming units", BRAZILIAN JOURNAL OF MICROBIOLOGY, vol. 44, no. 1, 1 January 2013 (2013-01-01), pages 287-290, XP055321599, BR ISSN: 1517-8382, DOI: 10.1590/S1517-83822013000100042

## Description

### FIELD OF THE INVENTION

The present invention concerns a bacterial detection method, in particular used to analyze biological samples in the laboratory.

More specifically, the present invention concerns a detection method particularly suitable for the detection of tuberculous bacteria, or bacteria that cause tubercular diseases.

### BACKGROUND OF THE INVENTION

It is known that tuberculosis (TB) is one of the diseases with the highest mortality in the world today. In 2013, nine million people contracted TB, 1.5 million died as a result of this disease, and 360,000 of these were also HIV-positive.

The threat of TB declined slowly in the years between 2000 and 2013, and it is estimated that 37 million lives have been saved thanks to an early diagnosis and specific treatment.

TB is present on all continents, although 80% of TB cases a year are recorded in a relatively low number of countries.

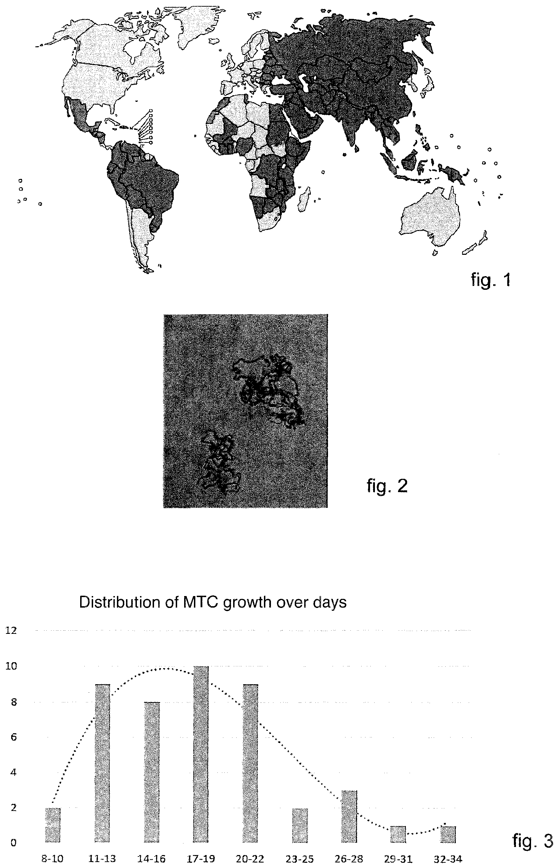

Of the 9 million people who developed TB in 2013, 56% of them live in regions of south-east Asia and the west of the Pacific (see the diagram in fig. 1, which shows the areas most affected by the disease in a darker shade of gray).

An additional 25% comes from Africa, which to date remains the area with the highest incidence of cases and deaths. India and China alone account for 24% and 11% of total cases respectively.

It is known that tuberculosis is transmitted by mycobacteria, called tuberculous mycobacteria (*Mycobacterium Tubercolosis* complex MTC) able to infect human beings through the air.

It is also known that only Mycobacterium Tubercolosis is responsible for the disease of tuberculosis, therefore the simple contagion from mycobacteria does not necessarily imply the transmission of the disease.

Non-tuberculous mycobacteria (MOTT - mycobacteria other than tuberculosis) have different replication times and morphologies compared with MTCs.

MTC pathogenesis is associated with the production of the cord factor, which is a molecule that is found in the cell wall of *Mycobacterium Tuberculosis,* and which provides the MTC with a characteristic serpentine appearance in liquid cultures.

Mycobacteria can generally be eliminated by using an antibiotic drug; however, over time, tuberculous mycobacteria have developed a high resistance against certain antibiotics (called multidrug resistant mycobacteria, MDR), and therefore, in these cases, it is first necessary to determine if the mycobacterium that has infected a human being is able to resist the drugs, and if so, which.

The World Health Organization (WHO) has highlighted the need to find a method to detect these mycobacteria quickly and clearly, in particular see the document *"*End TB Strategy. Global strategy and targets for tuberculosis prevention, care and control after 2015 (2014)".

At present, the technique to identify mycobacteria generally involves culture for very long periods (from a minimum of 42 days for liquid media up to 90 days for a Lowenstein Jensen medium test) and confirmation and antibiotic sensitivity tests.

These culture times make known techniques slow and expensive both in terms of energy and in economic terms.

One disadvantage of this method is that laboratories, in order to perform the confirmation and antibiotic sensitivity tests, have to handle the sample and therefore expose the mycobacterium to the external environment and therefore they must necessarily meet many, high safety requirements.

The laboratories therefore require experienced and qualified personnel, difficult to find in the most disadvantaged zones of the world: to date, out of 4.9 million cases notified in 2013, only 2.8 million of them, equal to 58%, have been diagnosed bacteriologically (from slide, culture or PCR test), while the remaining 42% were diagnosed only clinically.

Another disadvantage of known methods is given by the long times required for the confirmation and antibiotic sensitivity tests, which do not allow to administer any suitable antibiotic in a short time.

The articles by N. Hasegawa et al. "New Simple and Rapid test for Culture Confirmation...", by L.G. Wayne "Dynamics of Submerged Growth of Mycobacterium Tubercolosis..." and by Katia Penuelas-Urquides et al. "Measuring of Mycobacterium tuberculosis growth:..." disclose prior art examples for detecting Mycobacterium tuberculosis growth using optical systems.

Applicant has therefore set the target of overcoming the disadvantages of known methods to develop a bacterial detection method specifically focused on the identification of mycobacteria that cause the onset of tuberculosis or similar diseases.

The purpose of the present invention is therefore to develop an improved method to rapidly detect the presence of tuberculous mycobacteria inside biological samples, distinguishing them from MOTT bacterial species.

Another purpose of the present invention is to develop a method able to detect possible resistance to antibiotics of the mycobacteria possibly present, at the same time as their growth, thus preventing the many potentially dangerous and currently necessary manipulations.

Another purpose of the invention is to provide a method which is practical and does not require the handling of infected samples and exposing them to the external environment, consequently reducing the risk of transmission of the tuberculous mycobacterium.

Another purpose is to perfect a method that can also be used in laboratories where the level of safety is medium or low, that is, lower than Biosafety Level 3 BSL 3, and which can for example be carried out efficiently even in disadvantaged zones.

The Applicant has devised, tested and embodied the present invention to overcome the shortcomings of the state of the art and to obtain these and other purposes and advantages.

### SUMMARY OF THE INVENTION

The present invention is set forth and characterized in the independent claim, while the dependent claims describe other characteristics of the invention or variants to the main inventive idea.

In accordance with the above purposes, the present invention concerns a method to detect bacteria in a biological sample, wherein said method provides to determine if the biological sample comprises tuberculous mycobacteria (MTC) or non-tuberculous mycobacteria (MOTT).

According to one aspect of the present invention, the detection method provides to:
- subject the biological sample to a plurality of light scattering measurements in order to acquire a plurality of set of measurements as a function of time;
- process the set of measurements acquired, so as to determine at least the starting time of the exponential phase of bacterial growth, the maximum value of bacterial growth of the set of measurements acquired and the temporal development of at least one statistical parameter;
- calculate one or more characteristic values from the temporal development of the statistical parameter;
- analyze, by means of one or more algorithms making use of a neural network, at least the starting time of the exponential phase of bacterial growth, the maximum value of bacterial growth of the set of measurements acquired and the characteristic values calculated;
- determine, on the basis of the analysis performed by the neural network, if the biological sample comprises tuberculous mycobacteria (MTC) or non-tuberculous mycobacteria (MOTT).

According to the invention, the statistical parameter is a standard deviation .

According to possible embodiments, the method provides to determine the starting time of the exponential phase of bacterial growth when the average measurement of light scattering measurements of a group consisting of the last set of measurements acquired and of a number of sets of measurements prior to the last set of measurements acquired comprised between 1 and 12, preferably equal to 9, is greater than a predefined threshold value.

In this case the threshold value is defined in relation to the average value of the light scattering measurements of the first set of measurements acquired.

This determination allows to exclude non-significant isolated high values.

According to possible embodiments, the method provides to determine the starting time of the exponential phase of bacterial growth, comparing, as a function of time, the standard deviation of the light scattering measurements of the set of measurements acquired and verifying the moment after which the standard deviation increases more than a predefined threshold growth value.

The detection of the starting time of the exponential phase of bacterial growth allows to rapidly determine if a biological sample comprises mycobacteria or not, since the latter have a characteristic exponential growth time comprised in a defined temporal interval.

If the exponential bacterial growth occurs before a defined limit time, that is, if it occurs before the minimum time in which mycobacteria typically grow, the method provides to signal that the biological sample contains contaminants.

If no exponential bacterial growth is detected, the method provides to signal that the biological sample is negative.

If the biological sample contains contaminants or no bacterial growth has been detected, the method allows an operator to replace the biological sample and promptly perform a new test with another biological sample.

It is no longer necessary to wait for the acquisition of the entire curve of bacterial growth, but it is possible to quickly discriminate whether a sample has mycobacteria or not.

According to possible embodiments, the characteristic values are defined by the values of the progressive average of the temporal development of the standard deviation calculated starting from the starting time of the exponential phase of bacterial growth.

By progressive average calculated to a defined time t we mean the average value calculated from the starting time of the exponential phase of bacterial growth until the defined time t.

These characteristic values are advantageous, since they allow to determine with high precision if there are tuberculous mycobacteria (MTC) or non-tuberculous mycobacteria (MOTT) comprised in the biological sample.

According to possible embodiments, the biological sample is subjected to a plurality of light scattering measurements with a sampling frequency comprised between 0.5 kHz and 10 kHz.

According to possible embodiments, the biological sample is subjected to a plurality of light scattering measurements for a time comprised between 0.5 seconds and 5 seconds.

According to possible embodiments, the detection method provides that a

plurality of sets of measurements is acquired at defined intervals of time for a defined temporal period of at least up to about 11 days following the beginning of the exponential phase of bacterial growth.

According to possible embodiments, the method comprises at least a verification step in which it is determined if the tuberculous mycobacteria (MTC) in the sample are able to resist one or more antibiotic drugs, determining if the mycobacteria are the multidrug-resistant (MDR) type, or determining the single or crossed resistance at the same time as the bacterial growth.

According to possible embodiments, the method provides that the biological sample, once put in the culture, no longer comes into contact with the outside environment for the entire execution of the bacterial detection procedure.

According to possible embodiments, the method provides that the biological sample is inserted in a hermetically sealed container provided with a non-reversible closing device, that is, in a vial or other similar container able to seal the content and not allow any operator to open it.

### BRIEF DESCRIPTION OF THE DRAWINGS

These and other characteristics of the present invention will become apparent from the following description of some embodiments, given as a non-restrictive example with reference to the attached drawings wherein:
- fig. 1 is a diagram in which the areas of the world with the highest incidence of cases of tuberculosis are highlighted;
- fig. 2 is an enlargement under the microscope of a tubercular mycobacterium;
- fig. 3 is a bar diagram that indicates the distribution of the days of growth of tuberculous mycobacteria;
- fig. 4 is a graph that shows the radial distribution of the mycobacteria, either tubercular or not, in two clusters (A and B) using a K-means algorithm;
- fig. 5 schematically shows three sets of measurements acquired around the initial bacterial growth phase of the bacterial growth curve;
- figs. 6 and 7 show the temporal developments of the progressive average of the deviation standard of two biological samples respectively containing MTC and MOTT.

### DETAILED DESCRIPTION OF SOME EMBODIMENTS

We will now describe possible embodiments of a bacterial detection method in a biological sample, in which the method provides to determine whether the biological sample comprises tuberculous mycobacteria MTC or non-tuberculous mycobacteria MOTT.

The biological sample can be selected from one or more body fluids, such as respiratory fluids, pleural fluids, ascites, liquor, blood, bile, urine or suchlike, or it can be a different biological sample, for example feces, sputum, or other.

According to one aspect of the present invention, the detection method provides at least:
- to subject the biological sample to a plurality of light scattering measurements to acquire a plurality of sets of measurements as a function of time;
- to process the acquired set of measurements to determine at least the starting time of the exponential phase of bacterial growth, the value of the maximum bacterial growth of the acquired sets of measurements and the temporal development of at least one statistical parameter;
- to calculate one or more characteristic values of the temporal development of the statistical parameter;
- to analyze, by means of one or more algorithms that use a neural network, at least the starting time of the exponential phase of bacterial growth, the value of the maximum bacterial growth of the acquired sets of measurements and the characteristic values calculated;
- to determine, on the basis of this analysis performed by the neural network, whether the biological sample comprises tuberculous mycobacteria MTC or non-tuberculous mycobacteria MOTT.

According to possible solutions, the biological sample is put in a culture broth inside a container.

The culture broth can comprise a liquid phase Middlebrook broth, with the addition of PANTA additives (Polymyxin B, Amphotericin B, Nalidixic acid, Trimethoprim, Azlocillin), to which an OADC (Oleic Albumin Dextrose Catalase) culture supplement can be added.

In particular, it has been noted that with this solution the results regarding the screening culture phase are available from 24 to 48 hours before the MGIT system (Mycobacter Growth Indicator Tube), which is a market-leader instrument and known in the state of the art.

Mycobacteria in a liquid medium are oriented particles which, when subjected to a light generated by a source, disperse the light.

Depending on the response time and frequency of the refracted light, the method also allows to determine the size and morphological structure of the mycobacteria in the biological sample.

The present invention allows to determine one or more statistical parameters from the statistical distribution of the light scattering measurements acquired, from which it is possible to discriminate quickly and accurately whether the biological sample comprises tuberculous mycobacteria MTC or non-tuberculous mycobacteria MOTT.

The set of measurements consisting of the light scattering measurements will be characterized by a statistical distribution from which it is possible to extrapolate the standard deviation.

By subjecting the biological sample to a plurality of light scattering measurements at defined time intervals, it is possible to calculate the experimental points of the bacterial growth curve, each of the experimental points being defined by the median of the light scattering measurements acquired in a determinate time.

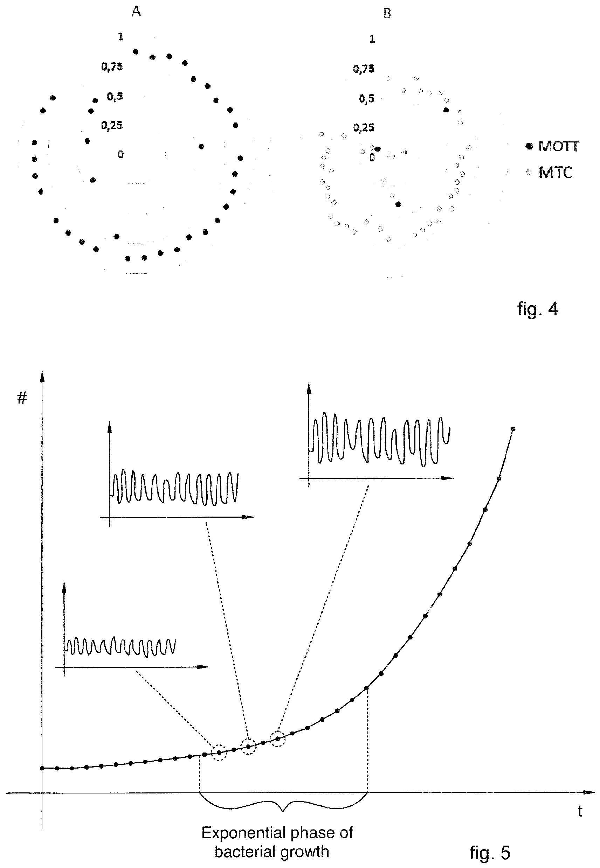

With reference to fig. 5, a bacterial growth curve is schematically shown, obtained from the detection of the experimental points defined by the median of the light scattering measurements acquired from the biological sample in a determinate time.

Each experimental point of the bacterial growth curve is obtained starting from a corresponding set of measurements consisting of the light scattering measurements acquired.

Fig. 5 shows how the sets of measurements acquired around the initial bacterial growth phase have statistical distributions useful for the detection and recognition of tuberculous mycobacteria MTC.

If the exponential bacterial growth occurs before a defined limit time, that is, if it occurs before the minimum time in which the mycobacteria typically grow, the method provides to signal that the biological sample contains contaminants.

According to possible embodiments, the time limit defined can be comprised between 2 and 4 days from when the culture is obtained, that is, from the beginning of the analysis, advantageously the time limit defined is 3 days from when the culture is obtained.

In the event that no exponential bacterial growth is detected, the method provides to signal that the biological sample is negative.

Applicant has identified how the statistical distributions and in particular the temporal development of the standard deviation of the sets of measurements allows to recognize if in the biological sample there are tuberculous mycobacteria MTC or non-tuberculous mycobacteria MOTT.

By processing the sets of measurements, or from their statistical distributions, it is possible to obtain characteristic values correlated to the morphology of the tuberculous mycobacteria.

According to possible embodiments, the characteristic values are defined by the values of the progressive average of the temporal development of the standard deviation calculated from the starting time of the exponential phase of bacterial growth.

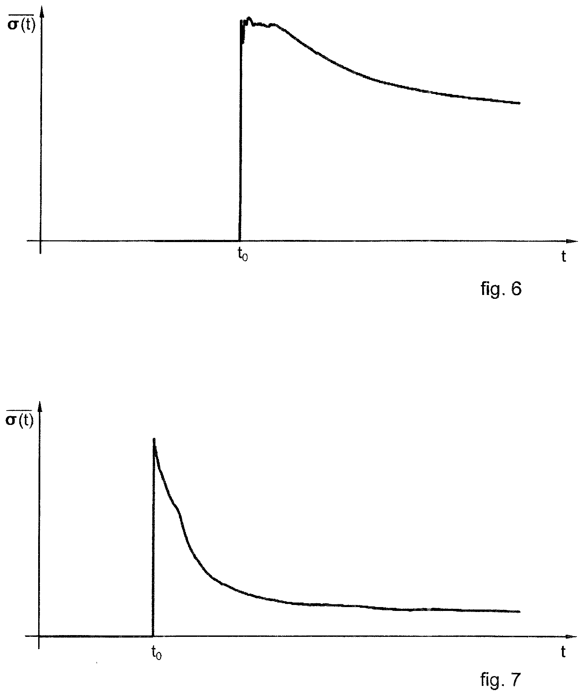

With reference to figs. 6 and 7 two temporal developments are shown of the progressive average of the standard deviations of two biological samples containing respectively MTC and MOTT.

As can be seen, the temporal development of the characteristic values of the sample with MTC are different from those relating to the MOTT sample.

From the differences in the two temporal developments it is possible to discriminate whether in a biological sample there are MTC or MOTT mycobacteria.

From the analysis of the characteristic values derived from the temporal development of the statistical parameter determined by the sets of measurements it is possible to determine, based on defined threshold values or using algorithms using a neural network, if the biological sample comprises tuberculous mycobacteria MTC or non-tuberculous mycobacteria MOTT.

Until now it has been known that in order to obtain information, or at least determine whether the biological sample comprises tuberculous mycobacteria MTC or non-tuberculous mycobacteria MOTT, it was necessary to have the bacterial growth curve in its entirety. This made known methods slow, with disadvantages not always admissible.

Applicant has identified a surprising link between the statistical distribution of the light scattering measurements and the morphology of tuberculous mycobacteria MTC which allows to quickly identify if the biological sample comprises tuberculous mycobacteria MTC by analyzing the set of measurements acquired.

In particular, a close link was identified between the function of autocorrelation of the set of measurements, as well as between the statistical parameters deriving from it, and the morphology of the tuberculous mycobacteria MTC.

In fact, due to the exclusive production of cord factor on the cell wall of such mycobacteria, their morphology and hence the statistical distribution of light scattering measurements allow to clearly distinguish these mycobacteria from MOTT.

Thanks to this, the detection method according to the present invention is able to provide a response much more quickly compared to the times necessary up to now.

Moreover, thanks to the present invention it is possible to determine, directly and without handling the potentially dangerous biological sample, whether it contains tuberculous mycobacteria MTC or non-tuberculous mycobacteria MOTT.

According to possible embodiments, the method provides that the biological sample is inserted in a hermetically sealed container provided with an irreversible closing device, that is, in a vial or other similar container able to seal the contents and not allow an operator to open it.

This guarantees a high level of safety for the operators, as the container, being provided with an irreversible closing device, cannot be opened once closed.

Unlike known techniques which use indirect methods, such as for example measurements of carbon dioxide or other gases, the present invention is highly sensitive.

Moreover, by acquiring and monitoring the light scattering measurements from the beginning, it is also possible to discriminate whether any possible contaminants are present in the sample, as they grow before the mycobacteria and with a different slope. In this case it will be possible to repeat the sampling of the biological sample and hence its analysis in a short time.

Furthermore, the method allows to quickly identify whether and how the mycobacteria grow in the expected time, without needing to acquire the entire bacterial growth curve.

According to possible embodiments, the plurality of light scattering measurements can be performed by means of an automatic scanning turbidimeter.

For example, vials with biological samples can be inserted into an automatic scanning turbidimeter able to simultaneously perform a plurality of light scattering measurements.

The turbidimeter can use laser light or other source of electromagnetic radiation. For example, the turbidimeter can be an HB&L-Uro4 system, produced and patented by Applicant and widely used in the sector.

According to possible embodiments, the processing of the set of measurements can be carried out by means of a processing unit able to determine at least the starting time of the exponential phase of bacterial growth, the maximum value of bacterial growth of the sets of measurements acquired and the temporal development of at least one statistical parameter.

The processing unit can also perform the calculation of the characteristic values from the temporal development, as well as the analysis using one or more algorithms using a neural network.

For example, the processing unit can be an electronic board, an integrated circuit or another similar or comparable microprocessor.

The processing unit can comprise, or be connected to, a calculator unit able to calculate one or more characteristic values from the temporal development of at least one statistical parameter.

The processing unit can comprise, or be connected to, an analysis unit able to process and execute one or more algorithms using a neural network to determine whether the biological sample comprises tuberculous mycobacteria MTC or non-tuberculous mycobacteria MOTT.

The analysis of the data is obtained with a neural network, in the case described here a known K-means algorithm, appropriately adapted for the purpose, by means of an analysis program.

According to possible variants, the analysis can be performed using algorithms based both on the K-Means algorithm and also on Self Organizing Maps.

According to possible embodiments, the biological sample is subjected to a plurality of light scattering measurements with a sampling frequency comprised between 0.5 kHz and 10 kHz.

According to possible embodiments, the biological sample is subjected to a plurality of light scattering measurements for a time comprised between 0.5 seconds and 5 seconds.

According to possible embodiments, the detection method requires that a plurality of sets of measurements is acquired at defined time intervals for a defined period of time at least up to about 11 days after the beginning of the exponential phase of bacterial growth.

This period of time allows to obtain a response with a high level of reliability, since by monitoring and analyzing the sets of measurements made according to the present invention it is possible to accurately identify whether the biological sample comprises tuberculous mycobacteria MTC.

According to possible embodiments, the method comprises at least a verification step in which it is determined whether the tuberculous mycobacteria MTC inside the sample are able to resist one or more antibiotic drugs, determining whether the mycobacteria are the multidrug-resistant mycobacteria type MDR, or determining single or crossed resistance simultaneously with bacterial growth.

By performing the antibiotic resistance tests simultaneously with the growth and detection step of the tuberculous mycobacteria MTC, it is possible to identify the most appropriate antibiotic therapy.

According to possible embodiments, the method provides that the biological sample in the culture does not come into contact with the external environment for the entire execution of the bacterial detection procedure.

In a possible application of the method according to the present invention, the biological sample was inoculated in two vials, each vial containing a culture broth. The vials can advantageously be provided with an irreversible closing device.

The first vial contains the biological sample, possibly pretreated in culture broth, while in the subsequent vial or vials, in addition to the biological sample, as in the first vial, one or more antibiotic drugs is also added.

Subsequent vials can comprise a single antibiotic to verify single sensitivity to a drug, or a cocktail of two or more different antibiotics to determine multi-resistance to antibiotics, for example cross-resistance to isoniazid/rifampicin.

If there is only one antibiotic per vial, using several vials containing different antibiotics, it is possible to analyze the sensitivity to the antibiotics SIRE (streptomycin, isoniazid, rifampicin, ethambutol).

The same method can possibly be extended to other antibiotics, for example pyrazinamide.

The use of antibiotics (individually tested or in cocktails) allows to better interpret which antibiotic or antibiotics are most suitable for health treatment.

By analyzing over a period of time, by means of light scattering techniques, the two or more test tubes under examination, it was possible to verify a growth phase of the mycobacteria (vial one) and, simultaneously with the sampling, if the strain had developed resistance to one or more MDR antibiotics, and if so to which (based on the corresponding vials).

To obtain the desired results, several measurements were performed during the day for several consecutive days, in order to evaluate the growth rate of the mycobacteria. In this case, the cultures were monitored at regular times, for example every 5 minutes or every 30 minutes for 6 weeks.

For the whole duration of the analysis, the vials remained sealed, completely eliminating the risk of airborne transmission of mycobacteria.

As we have seen, the bacterial detection method using analysis with a mathematical algorithm of data coming from light scattering tests is simple to use and provides clear results; moreover, the method described above is extremely safe and does not entail risks for the operator, as it does not expose the mycobacteria to the external environment, and therefore does not require the laboratory to meet BSL-3 safety standards.

In addition, the rapid identification of bacterial contamination allows the biological samples stored, if present, to be retested with minimal delay. Even more importantly, the parallel analysis of a second culture vial containing antibiotics (for example isoniazid and rifampicin), allows simultaneous phenotypic identification of MDR mycobacteria. In this way it also satisfies the requests made by the WHO, previously reported.

It is clear that modifications and/or additions of parts can be made to the bacterial detection method as described heretofore, without departing from the field and scope of the present invention, as described in the appended claims.

## Claims

1. Method to detect bacteria in a biological sample in order to determine if said biological sample comprises tuberculous mycobacteria (MTC) or non-tuberculous mycobacteria (MOTT), comprising:
- to subject said biological sample to a plurality of light scattering measurements in order to acquire a plurality of set of measurements as a function of time;
- to process said set of measurements acquired, so as to determine at least the starting time of the exponential phase of bacterial growth, the maximum value of bacterial growth of said set of measurements acquired and the temporal development of at least one statistical parameter;
- to calculate one or more characteristic values from said temporal development of said statistical parameter;
- to analyze, by means of one or more algorithms making use of a neural network, at least said starting time of the exponential phase of bacterial growth, said maximum value of bacterial growth of said set of measurements acquired and said calculated characteristic values, in order to determine if said biological sample comprises tuberculous mycobacteria (MTC) or non-tuberculous mycobacteria (MOTT),
wherein said statistical parameter is a standard deviation, wherein said characteristic values are defined by the values of the progressive average of said temporal development of the standard deviation calculated starting from said starting time of the exponential phase of bacterial growth.

2. Method as in claim 1, wherein said starting time of the exponential phase of bacterial growth is determined when the average measurement of said light scattering measurements of a group consisting of the last set of measurements acquired and of a number of sets of measurements prior to the last set of measurements acquired comprised between 1 and 12, preferably equal to 9, is greater than a predefined threshold value, and wherein the method provides to subject said biological sample to said plurality of light scattering measurements with a sampling frequency comprised between 0.5 kHz and 10 kHz and for a time comprised between 0.5 seconds and 5 seconds.

3. Method as in any claim hereinbefore, wherein, if the exponential bacterial growth occurs before a limit time comprised between 2 and 4 days, it provides to signal that the biological sample contains contaminants.

4. Method as in any claim hereinbefore, comprising to acquire said plurality of set of measurements at intervals of time defined for a temporal period defined at least up to about 11 days following the beginning of said exponential phase of bacterial growth.

5. Method as in any claim hereinbefore, comprising at least a verification step in which it is determined if said tuberculous mycobacteria (MTC) in said biological sample are able to resist one or more antibiotic drugs, determining if said mycobacteria are the multidrug-resistant (MDR) type, or determining the single or crossed resistance at the same time as the bacterial growth.

6. Method as in any claim hereinbefore, wherein said biological sample does not come into contact with the outside environment for the entire execution of the culture step and the bacterial detection procedure.

7. Method as in any claim hereinbefore, wherein said plurality of light scattering measurements is carried out by means of an automatic scan turbidimeter, and the processing, calculation and analysis are carried out by a processing unit able to determine said statistical parameters, to calculate said characteristic values from said at least one set of measurements and to determine if said biological sample comprises tuberculous mycobacteria (MTC) or non-tuberculous mycobacteria (MOTT).

## Patentansprüche

1. Verfahren zum Detektieren von Bakterien in einer biologischen Probe, um zu ermitteln, ob die biologische Probe tuberkulöse Mykobakterien (MTC) oder nicht-tuberkulöse Mykobakterien (MOTT) aufweist, aufweisend:
- Unterziehen der biologischen Probe einer Mehrzahl von Lichtstreumessungen, um eine Mehrzahl von Sätzen von Messungen in Abhängigkeit von der Zeit zu erfassen,
- Verarbeiten des erfassten Satzes von Messungen, um zumindest die Startzeit der exponentiellen Phase des Bakterienwachstums, den Maximalwert des Bakterienwachstums des erfassten Satzes von Messungen und die zeitliche Entwicklung von mindestens einem statistischen Parameter zu ermitteln,
- Berechnen von einem oder mehreren Kennwerten aus der zeitlichen Entwicklung des statistischen Parameters,
- Analysieren, mittels eines oder mehrerer Algorithmen, die ein neuronales Netz verwenden, zumindest der Startzeit der exponentiellen Phase des Bakterienwachstums, des Maximalwerts des Bakterienwachstums des erfassten Satzes von Messungen und der berechneten Kennwerte, um zu ermitteln, ob die biologische Probe tuberkulöse Mykobakterien (MTC) oder nicht-tuberkulöse Mykobakterien (MOTT) aufweist,
wobei der statistische Parameter eine Standardabweichung ist,
wobei die Kennwerte durch die Werte des progressiven Mittels der zeitlichen Entwicklung der Standardabweichung definiert sind, die ausgehend von der Startzeit der exponentiellen Phase des Bakterienwachstums berechnet wird.

2. Verfahren gemäß Anspruch 1, wobei die Startzeit der exponentiellen Phase des Bakterienwachstums ermittelt wird, wenn die durchschnittliche Messung der Lichtstreumessungen einer Gruppe, bestehend aus dem letzten Satz von erfassten Messungen und einer Anzahl von Sätzen von Messungen vor dem letzten Satz von erfassten Messungen, die zwischen 1 und 12 beträgt, vorzugsweise gleich 9 ist, größer als ein vordefinierter Schwellenwert ist, und wobei das Verfahren vorsieht, die biologische Probe der Mehrzahl von Lichtstreumessungen mit einer Abtastfrequenz zwischen 0,5 kHz und 10 kHz und für eine Zeit zwischen 0,5 Sekunden und 5 Sekunden zu unterziehen.

3. Verfahren gemäß irgendeinem vorhergehenden Anspruch, wobei, wenn das exponentielle bakterielle Wachstum vor einer Grenzzeit auftritt, die zwischen 2 und 4 Tagen beträgt, es vorsieht zu signalisieren, dass die biologische Probe Verunreinigungen enthält.

4. Verfahren gemäß irgendeinem vorhergehenden Anspruch, aufweisend das Erfassen der Mehrzahl von Sätzen von Messungen in Zeitintervallen, die für eine zeitliche Periode definiert sind, die als mindestens bis zu etwa 11 Tage nach dem Beginn der exponentiellen Phase des bakteriellen Wachstums definiert ist.

5. Verfahren gemäß irgendeinem vorhergehenden Anspruch, aufweisend mindestens einen Verifizierungsschritt, in dem ermittelt wird, ob die tuberkulösen Mykobakterien (MTC) in der biologischen Probe in der Lage sind, einem oder mehreren Antibiotika zu widerstehen, wobei ermittelt wird, ob die Mykobakterien vom multiarzneimittelresistenten (MDR) Typ sind, oder die Einzel- oder Kreuzresistenz gleichzeitig mit dem Bakterienwachstum ermittelt wird.

6. Verfahren gemäß irgendeinem vorhergehenden Anspruch, wobei die biologische Probe während der gesamten Durchführung des Kulturschrittes und des Bakteriendetektionsvorgangs nicht mit der äußeren Umgebung in Kontakt kommt.

7. Verfahren gemäß irgendeinem vorhergehenden Anspruch, wobei die Mehrzahl von Lichtstreumessungen mittels eines automatischen Abtast-Trübungsmessers durchgeführt wird und die Verarbeitung, Berechnung und Analyse durch eine Verarbeitungseinheit durchgeführt werden, die in der Lage ist, die statistischen Parameter zu ermitteln, die Kennwerte aus dem mindestens einen Satz von Messungen zu berechnen und zu ermitteln, ob die biologische Probe tuberkulöse Mykobakterien (MTC) oder nicht-tuberkulöse Mykobakterien (MOTT) aufweist.

## Revendications

1. Procédé de détection de bactéries dans un échantillon biologique afin de déterminer si ledit échantillon biologique comprend des mycobactéries tuberculeuses (MTC) ou des mycobactéries non tuberculeuses (MOTT), comprenant les étapes consistant à :
- soumettre ledit échantillon biologique à une pluralité de mesures de diffusion de lumière afin d'acquérir une pluralité d'ensembles de mesures en fonction du temps ;
- traiter ledit ensemble de mesures acquis, de manière à déterminer au moins l'instant de départ de la phase exponentielle de croissance bactérienne, la valeur maximale de croissance bactérienne dudit ensemble de mesures acquis et le développement temporel d'au moins un paramètre statistique ;
- calculer une ou plusieurs valeurs caractéristiques à partir dudit développement temporel dudit paramètre statistique ;
- analyser, au moyen d'un ou plusieurs algorithmes faisant appel à un réseau neuronal, au moins ledit instant de départ de la phase exponentielle de croissance bactérienne, ladite valeur maximale de croissance bactérienne dudit ensemble de mesures acquis et lesdites valeurs caractéristiques calculées, afin de déterminer si ledit échantillon biologique comprend des mycobactéries tuberculeuses (MTC) ou des mycobactéries non tuberculeuses (MOTT),
dans lequel ledit paramètre statistique est un écart type,
dans lequel lesdites valeurs caractéristiques sont définies par les valeurs de la moyenne progressive dudit développement temporel de l'écart type calculées à partir dudit instant de départ de la phase exponentielle de croissance bactérienne.

2. Procédé selon la revendication 1, dans lequel ledit instant de départ de la phase exponentielle de croissance bactérienne est déterminée lorsque la mesure moyenne desdites mesures de diffusion de la lumière d'un groupe constitué du dernier ensemble de mesures acquis et d'un nombre d'ensembles de mesures avant le dernier ensemble de mesures acquis compris entre 1 et 12, de préférence égal à 9, est supérieure à une valeur de seuil prédéfinie, et dans lequel le procédé prévoit de soumettre ledit échantillon biologique à ladite pluralité de mesures de diffusion de lumière avec une fréquence d'échantillonnage comprise entre 0,5 kHz et 10 kHz et pendant une durée comprise entre 0,5 seconde et 5 secondes.

3. Procédé selon l'une quelconque des revendications précédentes, dans lequel, si la croissance bactérienne intervient avant un temps limite compris entre 2 et 4 jours, il signale que l'échantillon biologique contient des contaminants.

4. Procédé selon l'une quelconque des revendications précédentes, comprenant l'étape consistant à acquérir ladite pluralité d'ensembles de mesures à des intervalles de temps définis pour une période temporelle définie au moins jusqu'à environ 11 jours après le début de ladite phase exponentielle de croissance bactérienne.

5. Procédé selon l'une quelconque des revendications précédentes, comprenant au moins une étape de vérification dans laquelle il est déterminé si lesdites mycobactéries tuberculeuses (MTC) dans ledit échantillon biologique sont capables de résister à un ou plusieurs antibiotiques, en déterminant si lesdites mycobactéries sont du type multipharmacorésistantes (MDR), ou
en déterminant la résistance unique ou croisée en même temps que la croissance bactérienne.

6. Procédé selon l'une quelconque des revendications précédentes, dans lequel ledit échantillon biologique ne vient pas en contact avec l'environnement extérieur pendant toute la durée de l'étape de culture et de la procédure de détection bactérienne.

7. Procédé selon l'une quelconque des revendications précédentes, dans lequel ladite pluralité de mesures de diffusion de lumière sont effectuées au moyen d'un turbidimètre à balayage automatique, et le traitement, le calcul et l'analyse sont effectués par une unité de traitement apte à déterminer lesdits paramètres statistiques, à calculer lesdites valeurs caractéristiques à partir dudit au moins un ensemble de mesures et à déterminer si ledit échantillon biologique comprend des mycobactéries tuberculeuses (MTC) ou des mycobactéries non tuberculeuses (MOTT).
